# EUROPEAN PATENT SPECIFICATION

(11) **EP 1 185 202 B1**
(45) Date of publication and mention of the grant of the patent: **29.11.2006**
(21) Application number: 00939791.0
(22) Date of filing: 12.06.2000
(51) Int. Cl.: A61B 10/00

(54) **ALIGNMENT DEVICES FOR FLUID EXTRACTION FROM TISSUE AND SUBSTANCE DELIVERY**
AUSRICHTUNGSVORRICHTUNGEN ZUR FLÜSSIGKEITSGEWINNUNG AUS GEWEBE UND ZUR SUBSTANZABGABE
DISPOSITIFS INTEGRES D'ALIGNEMENT, SYSTEMES DESTINES A L'EXTRACTION EFFICACE DE FLUIDE, A L'ADMINISTRATION DE SUBSTANCES ET AUTRES APPLICATIONS

(30) Priority: 11.06.1999 US 138738 P; 18.06.1999 US 140257 P; 26.05.2000 US 207677 P
(43) Date of publication of application: 13.03.2002
(73) Proprietor: SpectRx, Inc., Norcross, GA 30071 (US); Altea Therapeutics Corporation, Tucker, Georgia 30084 (US)
(72) Inventor: SAMUELS, Mark A., Norcross, GA 30092 (US); KUMAR, Krishna S., Tucker, GA 30084 (US); ROBINSON, Garrett T., Tucker, GA 30084 (US); FARQUHAR, J. David, Duluth, GA 30096 (US); CALAWAY, Allison, Atlanta, GA 30342 (US); WILLIAMS, Deidre, Atlanta, GA 30316 (US); HATCH, Michael R., Sugar Hill, GA 30518 (US); SMITH, Alan, Atlanta, GA 30305-2733 (US); WOODS, Teresa, Norcross, GA 30093 (US)
(74) Representative: Thomson, Paul Anthony
(86) International application number: PCT/US2000/016064
(87) International publication number: WO 2000/076575

(56) References cited:
- WO-A-90/04354
- WO-A-98/56293
- DE-A- 19 824 036
- US-A- 4 025 964
- US-A- 4 112 941
- US-A- 4 274 418
- US-A- 5 556 372
- US-A- 5 879 373
- US-A- 5 885 211

## Description

### BACKGROUND OF THE INVENTION

### Field of Invention

This invention relates to an alignment system and methods for aligning at least one apparatus with respect to a surface of a tissue by utilizing a tissue interface member and mating the apparatus to the tissue interface member during the operation of the apparatus. Furthermore, this invention could have direct application in any situation where accurate, repeatable repositioning of one object with respect to another is needed, specifically for positioning an object on the surface of a tissue in a repeatable manner. For example, the coupling of any type of sensor, monitor, or device (accelerometer, thermometer, pulse pressure monitor, electrode for sensing or delivering, etc.) could benefit from a reliable method of repositioning and guaranteed alignment. This invention may be used for either application of several of the same devices for comparison, or reapplication of the same device at prescribed intervals in time so long as the original tissue interface member can remain attached to the skin unaffected.

### Discussion of the Art

Techniques have been proposed for attaching measuring devices and delivery type devices to a patient. For example, U.S. Patent No. 4,274,418 describes a measuring device including an annular mounting member adapted to be fastened to the skin surface of a patient.

US-A-5 885 211 discloses tissue breaching devices having tissue interface members.

Previously, applications involving multiple or repeated engagement of an apparatus to a surface required hand-eye coordination for alignment. Often, this would lead to inaccurate alignment that would result in a less efficient and/or effective operation of the apparatus. The hand-eye coordination sometimes required a means for marking the desired location on the surface so as to use that marking as a reference point for subsequent alignment. However, this created a dependency on the operator that would lead to inconsistent results. In the field of continuous analyte monitoring of a biological tissue, oftentimes openings on the surface of the tissue are required to measure biological fluids. Techniques to create small openings in the tissue include the use of mechanical devices, thermal ablation and direct energy absorption. Where energy emitter devices are involved in the process, it is necessary to align the energy emitter device properly. For example, one thermal ablation technique creates openings utilizing a strip of energy absorbing film that is held in contact with the tissue. The film is responsive to energy directed thereon to heat up and to conductively transfer heat to the surface of the tissue to ablate the tissue. See, for example, U.S. Patent No. 5,885,211 for a further description of this thermal ablation technique.

Furthermore, in minimally invasive continuous analyte monitoring applications, the tissue ablation process creates openings to which vacuum can be applied to extract interstitial fluid or blood for measurement, or at which point a drug delivery device may be attached at the registration/poration site to deliver the desired drug through the openings. In situations where energy emissions are used to ablate the tissue, effective fluid collection, delivery and other handling processes can be hampered by the presence of the energy absorbing film. Moving the film out of the way for collection solves the interference problem, but then site registration for placement of the fluid extraction device and substance delivery device becomes an issue. This invention provides for a tissue interface member that maintains the desired alignment after removal of the dye layer so as to enable fluid extraction and substance delivery devices to operate at the desired registration site.

There is room for improving alignment methods, systems and devices where multiple apparatus and/or repeated apparatus application to a desired location on a surface is necessary and/or beneficial for effective use of an apparatus. Particularly in the area of continuous analyte monitoring, there exists a need to integrate and consolidate several functions of the analyte monitoring procedure into a single device. The present invention and its various embodiments accomplishes and satisfies this need by providing for an efficient means to make and maintain alignment of tissue breaching devices and sensors while also removing steps otherwise necessary for interfacing and operating those apparatus at the desired location on the surface of a tissue.

### SUMMARY OF THE INVENTION

The present invention is directed to an alignment system integrating a tissue interface member suitable for positioning at a desired location on the surface of the tissue and mating with an apparatus so as to maintain alignment of the apparatus during its operation as claimed in claim 1. This system can be used with various types of apparatus. For example, when applied in a continuous analyte monitoring system, the apparatus may be an energy emitter device commonly used to thermally ablate the surface of the tissue. Other types of apparatus that may be used include devices such as those utilizing mechanical or heated wire techniques. In addition, alignment of devices such as a sensor that measures analyte concentration or a drug delivery device is also an important part of a monitoring system.

Systems integrating the tissue interface member are also disclosed herein so that reliable and repeatable methods to properly center the desired apparatus may be applied. When applied to the field of continuous analyte monitoring, this integrated system allows for a poration mechanism to be applied and guarantees alignment as well as giving the user easy access to attach a device to the exposed adhesive site. In various embodiments of the invention, the tissue interface member adheres onto the skin and remains in its original position unaffected.

As will be evident by the following detailed description and the drawings herein, it will become apparent to one skilled in the art that the present invention and its various embodiments can be applied to numerous other systems for which alignment or repositioning at a specific centered location on a surface for continuous or numerous measurements is desired.

### BRIEF DESCRIPTION OF THE DRAWINGS

**Figure 1A** is a block diagram generally showing the environment of the alignment device according to the present invention.
**Figure 1B** is a block of an energy emitter apparatus having alignment features according to the present invention.
**Figure 1C** is a block diagram of an electrically heated element tissue breaching device having alignment features according to the present invention.
**Figure 1D** is a block diagram of a mechanical tissue breaching device having alignment features according to the present invention.
**Figure 1E** is a diagram of a fluid collection and sensor device having alignment features according to the present invention.
**Figure 2A** is a top view of a tissue interface member having biased clips according to one embodiment of the invention.
**Figure 2B** is a side view of the tissue interface member shown in **Figure 2A.**
**Figure 2C** is a view of the tissue interface member of the embodiment of the invention, as shown in **Figures 2A and 2B,** attached to an apparatus.
**Figure 3** illustrates the tissue interface member according to another embodiment of the invention, mating with an apparatus having a complementary threaded surface.
**Figure 4A** is a top view of tissue interface member and energy absorbing layer according to another embodiment of the present invention.
**Figures 4B and 4C** show the side view of elements of the embodiment of the invention shown in **Figure 4A.**
**Figures 4D and 4E** illustrate the embodiment of the inventions as shown in **Figures 4A-4C**, inclusively, as used in a continuous analyte monitoring system.
**Figure 5** is a perspective view of a tissue interface member and a portion of an apparatus that mates thereto, according to another embodiment of the invention.
**Figure 6** is a side view of the tissue interface member according to another embodiment of the invention and mating with another apparatus.
**Figure 7A** is a top view of tissue interface member according to still another embodiment of the invention.
**Figure 7B** is a side view of the tissue interface member shown in **Figure 7A.**
**Figures 8A through 8G** are side views showing operation steps of a tissue interface member used as part of a continuous analyte monitoring system.
**Figure 9** is a diagram of a tissue interface member and an energy emitter device and illustrating a control activation feature according an embodiment of the present invention.

### DETAILED DESCRIPTION OF THE INVENTION

### Definitions

As used herein, the term "biological membrane" means the structure separating one area of an organism from another area of the organism, such as a capillary wall, or the outer layer of an organism which separates the organism from its external environment, such as skin, buccal mucosa or other mucous membrane. The term "epithelial tissue, " when used herein is mean to mean skin, mucosa and linings of the body cavities of an organism.

As used herein, the term "tissue" means an aggregate of cells of a particular kind, together with their intercellular substance, that forms a structural material. The preferred tissue is the skin; however, other tissues suitable for use with this invention include mucosal tissue and soft organs. These examples, as are other examples used throughout this specification, are for illustrative purposes only and are not intended to be inclusive of all possibilities or suitable uses.

As used herein, the term "suction" or "pressure" relates to the relative pressure as compared to the internal pressure of the organism to which the system is interfaced. "Vacuum" is used synonymously with the term "suction."

As used herein, "ablation" refers to the process of controlled removal of a selected area of tissue from the surrounding tissue by kinetic energy released when the temperature of vaporizable substances in the selected area is rapidly elevated above the vaporization point thereby flash vaporizing some of the tissue in the selected area.

As used herein, the term "biological fluid" means blood serum, whole blood, interstitial fluid, lymph fluid, spinal fluid, plasma or any combination of these fluids. "Interstitial fluid" means the clear fluid that occupies the space between the cells in the body.

As used herein, "poration," "microporation," or any such similar term means the artificial formation of a small hole, opening or pore to a desired depth in or through a biological membrane, such as skin or mucous membrane, or the outer layer of an organism to lessen the barrier properties of this biological membrane to the passage of biological fluids, such as analytes from within the biological membrane or the passage of permeants or drugs from without the biological membrane into the body for selected purposes, or for certain medical or surgical procedures. The size of the hole or "micropore" so formed is approximately 1-1000µm in diameter. It is to be understood that the term "micropore" is used in the singular form for simplicity, but that multiple openings or pores may be formed by the integrated device according to the present invention.

As used herein, "opening" means any physical breach of the biological membrane of a suitable size for delivering or extraction fluid therethrough, including, but not limited to, micropores.

The term "porating element" is meant to include any means of forming a micropore, hole or opening described above, including by thermal ablation, mechanically breaching the tissue by lancet or needle, and other known techniques. Several types of tissue breaching techniques, including thermal ablation methods, are disclosed in U.S. Patent No. 5,885,211. An example of a mechanical porator device is disclosed in commonly assigned published PCT Application WO 9800193, entitled, "Multiple Mechanical Microporation Of Skin Or Mucosa." Another porating technique suitable for use in connection with this system is disclosed in commonly assigned PCT Application No. PCT/US99/15967 now published as EP-A-1 124 607, entitled "Controlled Removal Of Biological Membrane By Pyrotechnic Charge For Transmembrane Transport," filed July 14, 1999.

The term "heated probe" or "heat conducting element" means a probe, preferably solid phase, which is capable of being heated in response to the application of electrical, mechanical, sonic, magnetic, electromagnetic or optical energy thereto for achieving thermal ablation of the tissue. For simplicity, the probe is referred to as a "heated probe" or "heatable probe" which includes a probe in a heated or unheated state, but which is heatable.

The term "continuously" when used in connection with a continuous analyte monitoring system, means acting on an ongoing basis at a frequency or event rate that may vary depending on a particular application of the system. For example, the output of the sensor may be read on a periodic basis, such as every minute, several minutes, hour, several hours, etc. Moreover, at each reading event the sensor output is optionally sampled multiple times so as to obtain a plurality of readings relatively close in time whereby an average or other adjustment of those multiple readings is made for determining a final reading that is displayed or logged. An example of a continuous monitoring system is disclosed in PCT Application No. PCT/US99/16378, filed July 20, 1999, now published as WO-A 0 004 832 and entitled System and Method for Continuous Analyte Monitoring.

The term "apparatus" means tissue breaching devices, such as an energy emitter device (laser), micro-lancets, micro-needles, and other mechanical tissue breaching devices, an electrically heated element device for performing thermal ablation as disclosed in U.S. Patent No. 5,885,211, a sensor device such as an analyte sensor (glucose, etc.), and a drug delivery device, or any other type of device used to interface with a surface of the biological tissue for the desired operation of the device.

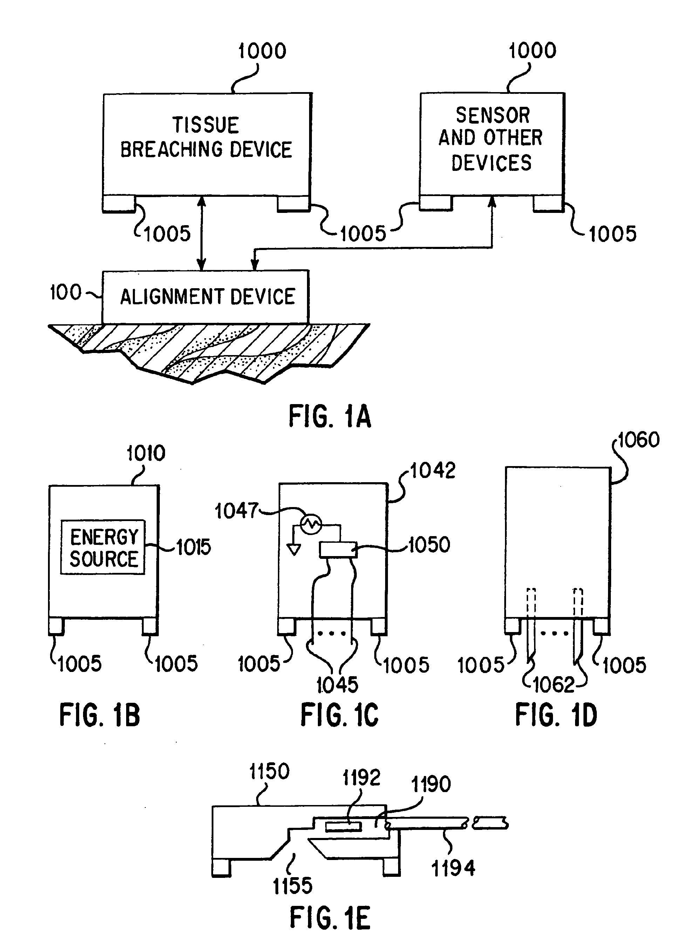

The present invention is directed to an alignment device suitable for positioning on the surface of the tissue, preferably at a desired location on the surface of the tissue, and to systems and methods for using the alignment device. Referring to Figure 1A, the alignment device, shown generally at 100, is positioned, attached or placed on the surface of a tissue, such as skin. The alignment device 100 mates with apparatus 1000 that may be one of a variety of tissue breaching devices, sensors, etc. The apparatus 1000 include at least one alignment member 1005 that mates or engages with complementary alignment members of the alignment device 100.

Figures 1B through 1E illustrate examples of the various types of apparatus 1000 that mate with the alignment device 100, all of which may include any one or more of the specific alignment structures disclosed hereinafter. Figure 1B illustrates an energy emitter device 1010 comprising at least one energy source 1015, such as a laser. An example of a suitable laser device is disclosed in U.S. Patent No. 5,885,211. The energy source 1015 may be a type that is used together with an energy absorbing material, such as an optical energy absorbing dye film, to ablate tissue by thermal ablation. Alternatively, the energy source 1015 may be a type that is used to cause the direct absorption of energy to ablate the tissue. In either case, alignment to the tissue surface is achieved by providing at least one alignment member 1005 on the energy emitter apparatus that mates with the alignment device 100.

Figure 1C shows a heated element tissue breaching device 1042 comprising one or more electrically heatable elements 1045. Electrical current is supplied to the heatable elements 1045 from a current source 1047 under control of a controller 1050. Further details of the device 1042 are disclosed in U.S. Patent No. 5,885,211 and in PCT Application No. PCT/US99/04990, now published as EP-A-1 059 883, filed March 5, 1999. The device 1042 includes at least one alignment member 1005 to mate with the alignment device 100 and thereby properly aligns the elements 1045 with the tissue surface via the alignment device 100.

Figure 1D illustrates a mechanical tissue breaching device 1060 comprising at least one tissue piercing element 1062, such as a micro-lancet or micro-needle. The device 1060 has at least one alignment member 1005 to mate with the alignment device 100 and properly align the tissue piercing element 1062 with the tissue surface. The tissue piercing element 1062 may be retracted in the device 1060 when not in use, and released into the tissue by one of a variety of mechanisms known in the art, such as those used in glucose test kits. Alternatively, the device 1060 may comprise a plurality of tissue penetrating members fabricated using micro-lithographic techniques as described in aforementioned PCT Application No. WO 9800193.

Figure 1E illustrates basic components of a fluid collection and sensor device 1150. The device 1150 has at least one alignment member 1005 to mate with the alignment device 100 in order to position a harvesting head or opening 1155 with openings made in the tissue beneath the alignment device. The fluid collection and sensor device 1150 further comprises an assay element 1192 positioned in or proximate a fluid collection chamber 1190. The assay element 1192 is responsive to one or more substances in the fluid collected from the tissue, such as glucose. Fluid from the tissue is drawn into contact with the assay element 1192 under application of vacuum supplied via a cable 1194. The details of a suitable fluid collection and sensor device are disclosed in PCT Application Nos. PCT/US00/09393, filed April 7, 2000, PCT/US99/16226, filed July 20, 1999, and PCT/US99/16378, filed July 20, 1999, now published as EP-A-1 164 925, EP-A-1 098 589 and EP-A-1 098 594.

Examples of other apparatus include monitors, thermometers, pulse pressure monitors, accelerometers, sensing or stimulating electrodes, etc. Regardless of the type of apparatus used, the present invention provides a means for repeatable, reliable and guaranteed alignment at the desired position to which the alignment device may be attached.

In various embodiments, the present invention is described as being useful in continuous analyte monitoring. In such instances, the present invention allows for a reliable and repeatable method to properly center a fluid harvesting device (also called a fluid collection and sensor device). This integrated system allows for a tissue breaching device to be applied and guarantees alignment as well as giving easy access to attach a device to the exposed site. The tissue interface member can adhere to the tissue and remain in its original position unaffected. Although various embodiments of the present invention are directed towards continuous analyte monitoring, it will become apparent to one skilled in the art that the present invention could be used with various applications to other uses that require alignment with a specific centered location on the surface of a tissue for continuous or numerous measurements, applying therapies of any variety, and creating openings in the tissue of any size, etc.

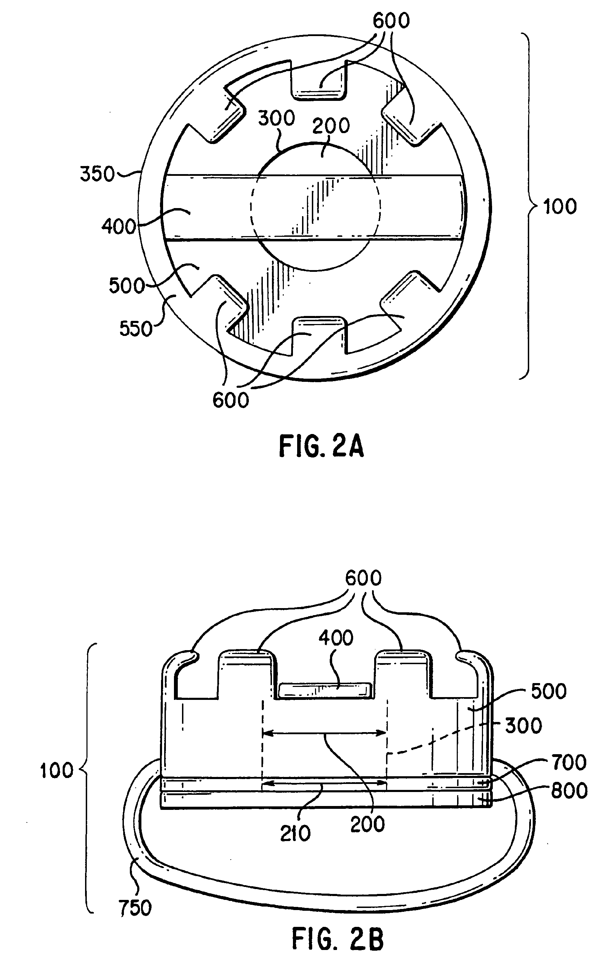

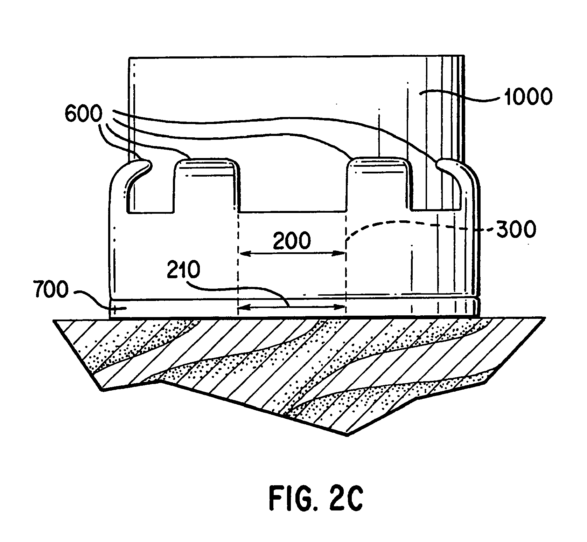

According to one embodiment of the present invention, Figures 2A and 2B show the alignment device 100 comprising a tissue interface member 500 having a raised perimeter 550 along the circumference thereof with at least one clip 600 extending therefrom. The clip 600 is biased by virtue of its inwardly curved lip or other structural feature (known in the art of mechanical clip design) so that it engages an apparatus inserted therein and holds it in engagement with the tissue interface member 500. Furthermore, the tissue interface member 500 has an opening or passageway 200 circumscribed by an interior surface 300 of the tissue interface member 500. With reference to Figure 2C, when an apparatus 1000 is properly inserted into the tissue interface member 500 and snapped into place beneath the clip(s) 600, the tissue interface member 500 holds the apparatus 100 in a predetermined or desired relationship with respect to the opening 200, and thus with a tissue surface underlying the opening as shown in Figure 2C. This allows the apparatus 1000 to interact with the surface of the tissue at the desired location maintained by the alignment device 100. Figures 2A-2C are enlarged and are not to scale (particularly as to the thickness of the device) in order to illustrate the various structural features of the alignment device.

When the tissue breaching device involves an energy emitter apparatus, oftentimes energy absorbing film is used therewith. The film is responsive to energy directed thereon to heat up and to conductively transfer heat to the surface of the tissue to ablate the tissue. Such an optical thermal ablation process is disclosed in aforementioned U.S. Patent No. 5,885,211. Referring back to Figure 2A in conjunction with Figure 2B, an energy absorbent layer 400 is shown placed across the top of the opening 200 of the tissue interface member 500. An adhesive layer 700 and a release liner 800 are provided on a bottom surface of the tissue interface member 500 for attaching the alignment device 100 to the surface of a tissue. When the alignment device 100 is attached via the adhesive layer 700, the release liner 800 is first removed so that the adhesive layer 700 may be exposed and attached to the desired location on a surface. The adhesive layer 700 also has an opening or passageway 210 therein circumscribed by the interior 305 of the adhesive layer 710. Moreover, the adhesive layer opening 210 is in alignment with the opening 200 of the tissue interface member 500. In the alternative or in combination, the alignment device 100 can further comprise a strap 750 that attaches to the tissue interface member 500 and extends around a body portion of an user, such as an arm, leg, or waist, so as to mount and hold the tissue interface member 500 at the desired location on the surface of the tissue for the desired duration of time.

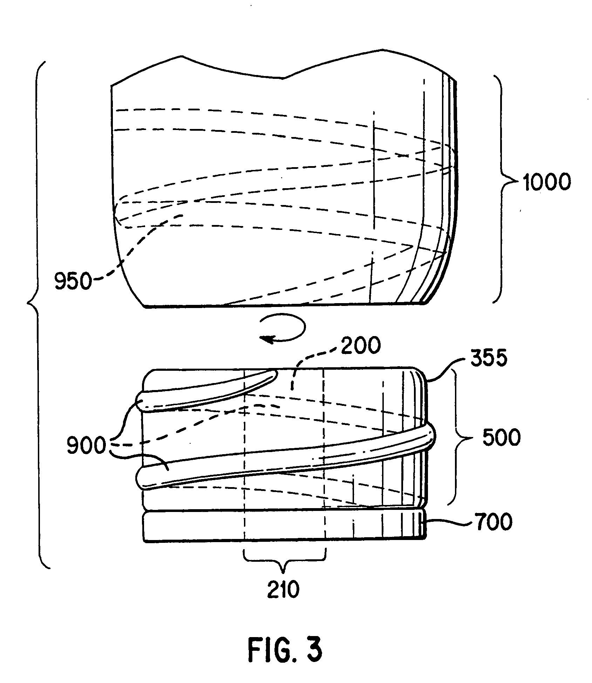

According to another embodiment shown in Figure 3, the tissue interface member 500 may engage with an apparatus via a threaded member 900 that circumscribes a side exterior surface 355. Like Figures 2A-2C, Figure 3 is not drawn to scale in order to best illustrate the invention. The opening 200 longitudinally traverses through the tissue interface member 500 and is aligned with the adhesive layer opening 210 of the optional adhesive layer 700. Figure 2 illustrates how a surface of an apparatus 1000 has a complementary threaded member 950 therein that mates with the threaded member 900 of the tissue interface member 500.

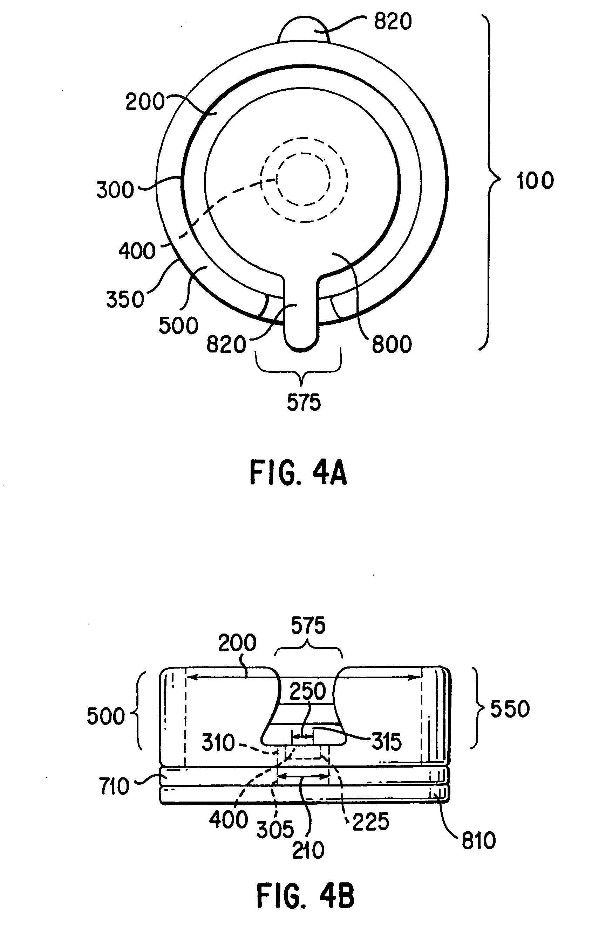

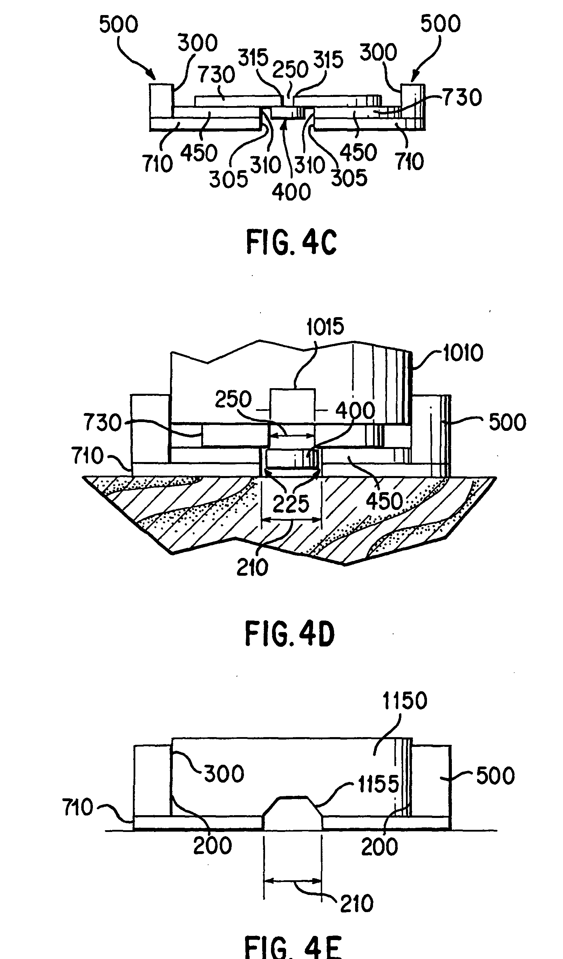

Figures 4A - 4E are directed to another embodiment of an integrated alignment device according to the present invention. In this embodiment, the integrated alignment device 100 is designed for an application that involves the use of an energy emitter device and an energy absorbent layer 400, in cooperative operation, to ablate the surface of a tissue. The alignment device 100 comprises a tissue interface member 500 that is circular in shape with an opening 200 therein and several layers attached thereto to facilitate placement on the surface of the tissue and engagement of various apparatus.

As shown in Figure 4B, several release liner/adhesive layers are in a sandwich-type configuration. There is a bottom double-sided adhesive layer 710 attached on its top side to the bottom of the tissue interface member 500 and covered on its bottom side by a bottom release liner 810. This bottom release liner 810 may be removed so that the bottom adhesive layer 710 and the tissue interface member 500 may adhere to the skin. The bottom adhesive layer 710 preferably is one that is not irritable, toxic or otherwise hazardous to the skin but is strong in its adhesiveness to allow the tissue interface member 500 to remain attached to the surface of the skin when used with multiple applications of an apparatus to the tissue interface member 500. An example of such type of an adhesive commonly used is the Brandon 2656B double adhesive. The bottom adhesive layer 710 also has an opening 210 therein that is circumscribed by the interior surface 305 of the bottom adhesive layer 710. This adhesive opening 210 is smaller in diameter than the diameter of the tissue interface member 500. The tissue interface member 500 is attached along the perimeter of the top side of the bottom adhesive layer 710. Above the bottom adhesive layer 710 and within the tissue interface member 500, there is a carrier layer (not shown) with an aperture 225 that contains an energy absorbent layer 400 therein aligned with the adhesive opening 210. A pocket or gap 575 is provided to allow room for a cable that may connect to an apparatus that mates with the tissue interface member shown in these diagrams. The internal elements of the tissue interface member 500 are better shown in Figure 4C.

Turning to Figure 4C, the area of the top side of the bottom adhesive layer 710 circumscribed by the interior surface 300 of the tissue interface member 500 is attached to the non-sticky (e.g. silicon) surface of a carrier layer 450. An example of a carrier layer that may be used is the Kraft Release Liner with a silicon surface on one side and a paper surface on the other side. The carrier layer 450 also has a carrier layer aperture 225 therein circumscribed by the interior surface 310 of the carrier layer 450. The carrier layer aperture 225 is in alignment with the adhesive opening 210 and may be the same size or smaller. Within the carrier layer aperture 225 lies an energy absorbent layer 400 that is concentric to but smaller than the carrier layer aperture 225 and in alignment with the carrier layer aperture 225 and the adhesive opening 210. The energy absorbent layer 400 is fixed in its position within the carrier layer aperture 225 by the bottom side of a top double adhesive layer 730. The top double-sided adhesive layer 730 also fits within the opening 200 circumscribed by the interior surface 300 of the tissue interface member 500. Furthermore, the top double-sided adhesive layer 730 has an orifice 250 therein that is also circumscribed by the interior surface 315 of the top double-sided adhesive layer 730. The orifice 250 is concentric to but smaller than the carrier layer aperture 225 and again is in alignment with the carrier layer aperture 225 and the adhesive opening 210. The size and alignment of the orifice 250 allows the top double-sided adhesive layer 730 to circumscribe and overlap the interior perimeter of the carrier layer aperture 225. This overlap provides the surface area to which the energy absorbent dye layer 400 may attach enabling it to be fixed in such a position so that it suspends with the carrier layer aperture 225. Finally, a non-sticky side of the top release liner 830 attaches to the top of the top double-sided adhesive layer 730 until the alignment device 100 is ready to be used. Similar to the bottom release liner 810, the top release liner 830, as shown in Figure 4A, also has an extended flap portion 820 for the user to grab to facilitate the removal of the release liner.

Figures 4D and 4E show how the alignment device of Figures 4A-4C is used in a continual analyte monitoring system. Once the bottom release liner 810 is removed, the tissue interface member is attached to the surface of the tissue (skin) via the bottom adhesive layer 710. The top release liner 830 is then removed and an energy emitter device 1010 (such as a laser diode apparatus) is inserted into the tissue interface member 500 and engages the top double-side adhesive layer 730. The tissue interface member 500 is already attached to the surface of the tissue via the bottom adhesive layer 710. When in position in the tissue interface member 500, the energy emitter device 1010 is aligned such that at least one source of an energy emission 1015 emitted by the energy emitter device 1015 is in alignment with the orifice 250 which is in alignment with the energy absorbent layer 400, which is in alignment with the carrier layer aperture 225, which in turn is in alignment with the adhesive opening 210 as shown in Figure 4D. After the energy emission is complete, the energy emitter device 1010 can then be removed from the tissue interface member 500 leaving the integrated alignment device 100 fixed at the original alignment registration site. According to this embodiment of the invention, removal of the energy emitter device 1010 also simultaneously removes top adhesive layer 730, the energy absorbent layer 400 and the carrier layer 450 in one step, leaving the tissue interface member 500 attached to the surface of the skin by the bottom adhesive layer 710. Referring to Figure 4E, the harvesting head 1155 of the fluid collection and sensor device 1150 may be inserted into and mated with the tissue interface member 500 which aligns the harvesting head 1155 with affected site of the tissue so that the source of suction is directly over the adhesive opening 210 and over the affected tissue site (not shown) created by the previous application of the energy emitter device.

The selection of materials and dimensions of an alignment device according to the present invention may vary with the particular application. In the case where the energy absorbing layer 400 is used in connection with a laser diode type energy emitter device, the energy absorbing layer is formed of a layer of PET (1 mil) and of Acetylene Black (2 mil) and approximately 4.9 mm in diameter. The thickness of the top adhesive layer 730 is 6.3 mil and the thickness of the bottom adhesive layer 710 is 6.0 mil. The diameter of the orifice 250 is 3.5 mm and the diameter of the opening 210 is 5.0 mm.

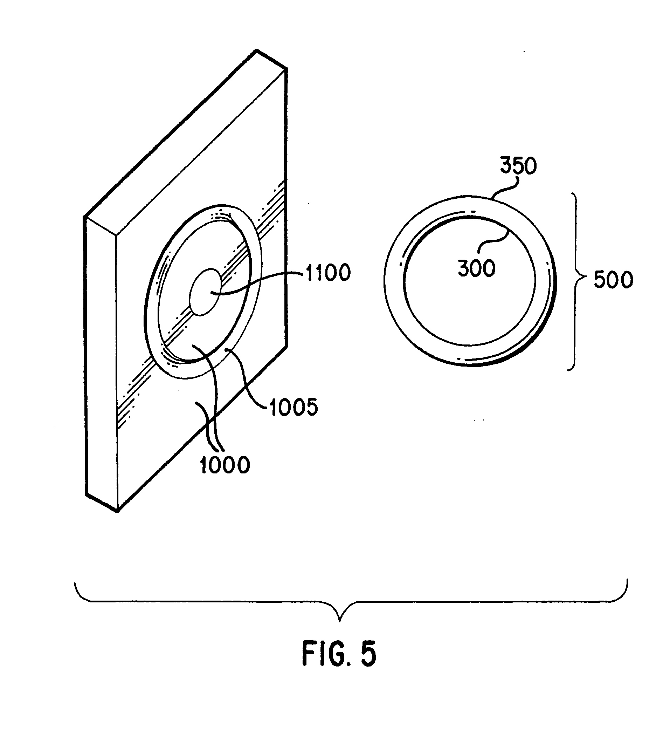

Other embodiments of the invention provide for various other means for which the tissue interface member might engage with an apparatus. For example, the tissue interface member can comprise any planar geometric shape, such as a triangle, a circle, ellipse, rectangle, etc., to facilitate interface with an apparatus that contains complementary elements to mate with the tissue interface member. Figure 5 shows an embodiment where the tissue interface member 500 comprises a circular shape with an opening 200 therein circumscribed by its interior surface 300. According to this embodiment, the interior surface 300 and the exterior surface 350 of the tissue interface member 500 mate to a complementary shaped groove or indented region 1005 of a tissue interface member engaging portion of the apparatus 1000.

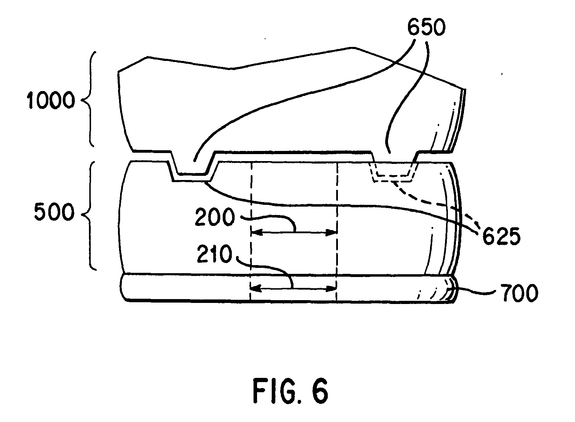

In addition or in the alternative to any of the embodiments described herein, the tissue interface member can have additional structural features that facilitate mating with an apparatus. Examples of such characteristics include, but are not limited to, complementary magnetic surface portions, adhesive on engaging surfaces, and/or complementary male or female members. For example, Figure 6 shows a tissue interface member that has at least one female member 625. According to this embodiment, the tissue interface member engaging portion of an apparatus 1000 has complementary male member(s) 650 that mate with the female members 625 of the tissue interface member 500 to achieve and maintain the desired alignment while the tissue interface member 500 is attached to the tissue surface. Furthermore, these male or female members can also have complementary magnetic surfaces or adhesive to enhance attachment and maintenance of the alignment between the tissue interface member 500 and the tissue interface member engaging portion of the apparatus 1000.

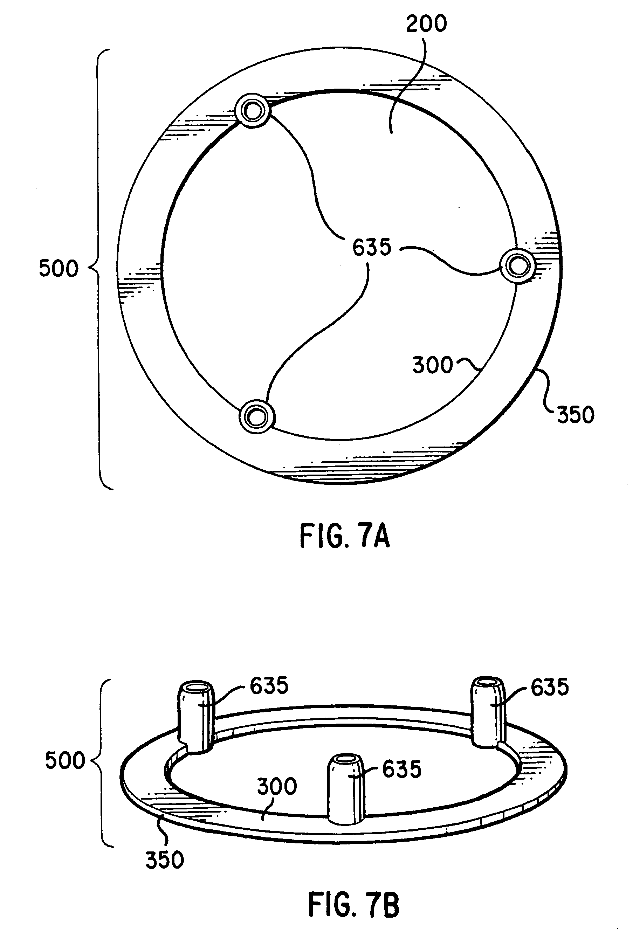

Figures 7A and 7B illustrate another embodiment of the invention where the top surface of the tissue interface member 500 comprises of at least one male member 635 and also has an opening 200 circumscribed by the interior surface 300 so that an apparatus may interact with the surface of the tissue via opening 200. Complementary female members would be on the apparatus that are designed to mate with the tissue interface member shown in Figures 7A and 7B.

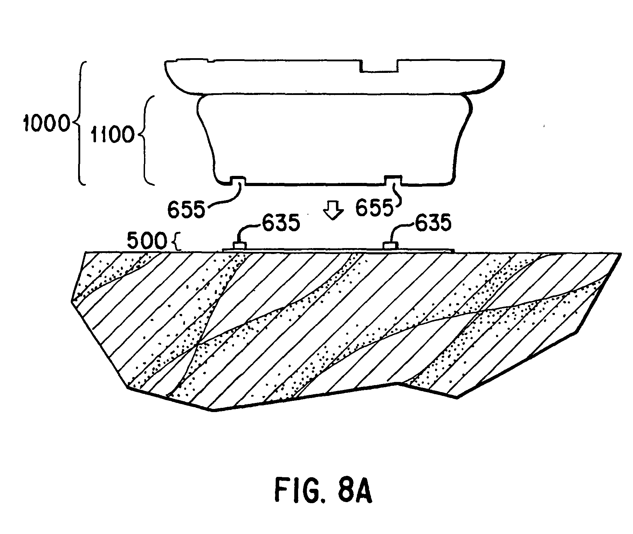

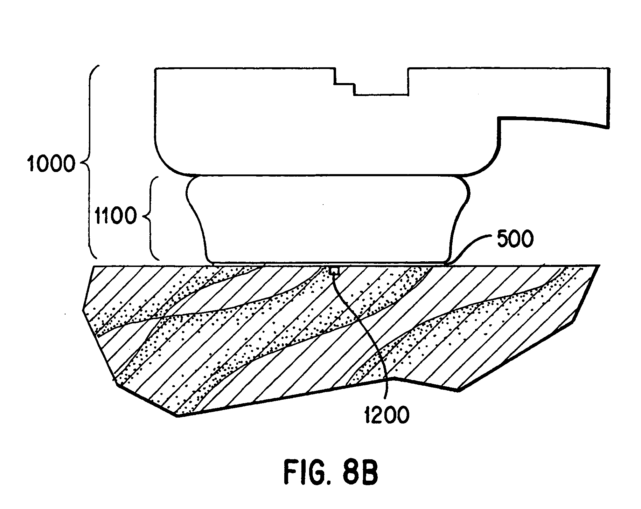

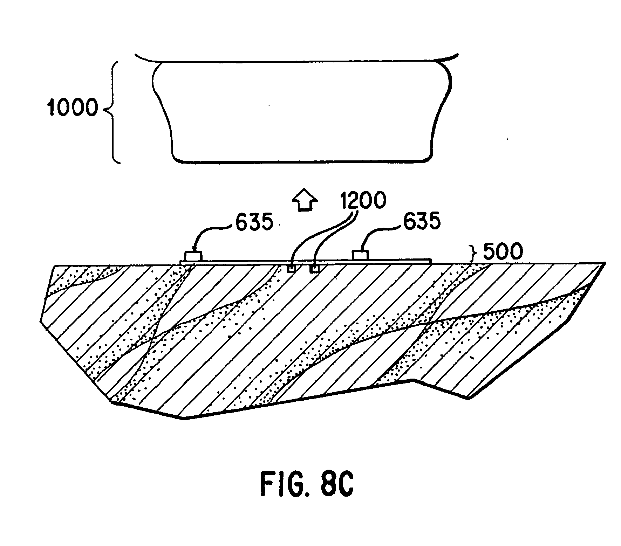

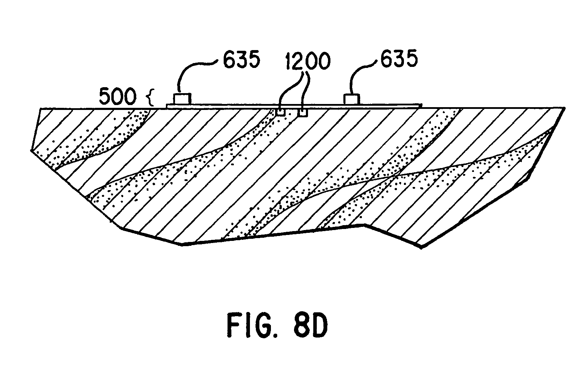

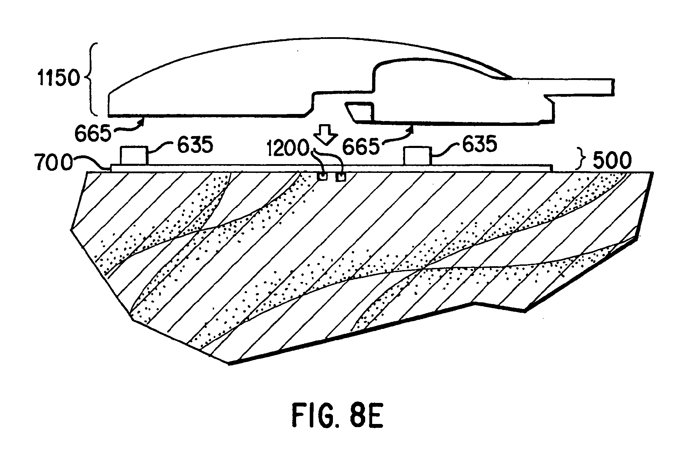

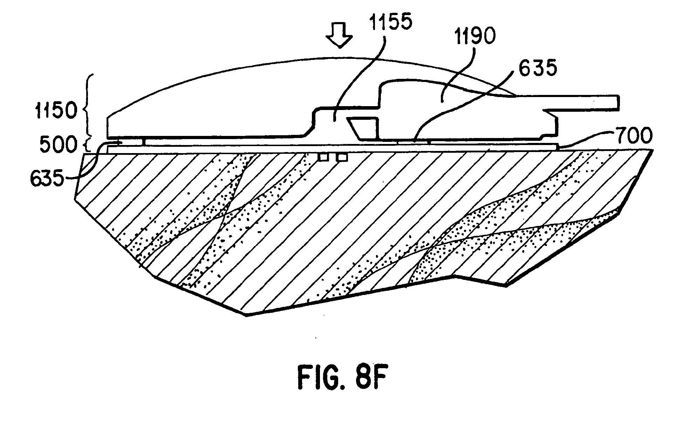

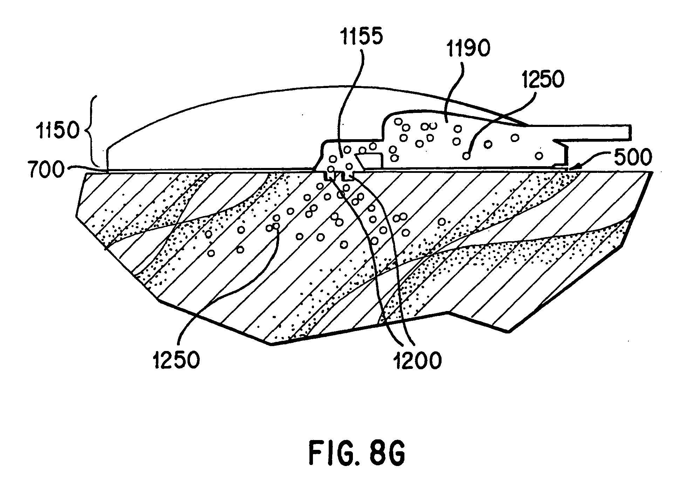

Figures 8A - 8G inclusively show operation of an alignment device according to one embodiment of the invention (Figures 7A and 7B) in the context of a continual analyte monitoring system. It should be understood that the alignment device according to the other embodiments operates in a similar fashion according to its structural features. Figure 8A shows the tissue interface member 500 attached to the surface of a skin via an adhesive (not shown). The tissue interface member 500 has at least one male member 635 as shown in Figures 7A and 7B. A tissue interface member engaging portion 1100 of an apparatus 1000 (in this case a tissue breaching device) with at least one complementary female member 655 is placed above the tissue interface member 500. Figure 8B shows the tissue breaching apparatus 1100 mating with the tissue interface member 500 via their complementary male and female members, respectively. Figure 8B also shows that the tissue breaching device 1100 has formed at least one opening 1200 through the surface of the tissue. The manner in which these openings are formed depends on the type of tissue breaching apparatus selected (mechanically piercing the tissue, thermally ablating the tissue with an electrically heated wire, thermally ablating the tissue by heating an energy absorbing layer in contact with the tissue with a beam or field of energy, emitting a beam or field of energy that is directly absorbed by the tissue to form the openings, etc.) An example of an energy emitter apparatus is an laser beam device disclosed in U.S. Provisional Applications No. 60/140,003, filed June 18, 1999 and 60/165,814, filed November 16, 1999 (WO-A-0 078 242 and WO-A-0 078 242). Figure 8C shows the tissue interface engaging portion 1100 of the tissue breaching device (not shown) detaching from the tissue interface member 500 after creating at least one opening 1200 on the surface of the tissue. The tissue interface member 500 remains attached to the surface of the tissue at the initial registration site. Figure 8D is another view of the tissue interface member 500 remaining fixed at the original placement site after the surface of the tissue had been breached by a tissue breaching device. Figure 8E shows the fluid collection and sensor device 1150 having complementary female members (their general location being shown by arrows 665 but not in view in Figure 8E) that mate to male members 635 on the tissue interface member 500. The male members 635 on the tissue interface member are in a fixed and known position such that the openings 1200 formed in the tissue by the tissue breaching device are at a fixed position with respect to the tissue interface member 500. Consequently, the subsequent attachment of the fluid collection and sensor device 1150 to the tissue interface member (with female members 665 placed at a fixed and known position with respect to internal structures thereof) will achieve proper alignment with the openings 1200 to draw fluid (by vacuum) from the openings into the harvesting head 1155 (which is essentially an opening into a housing of the sensor device 1150) where the fluid collection/analysis chamber 1190 is located inside the sensor device 1150. This interaction is facilitated and enhanced by the consistent registration to the site by the tissue interface member 500. Figure 8F shows the fluid collection and sensor device 1150 matingly engaging the tissue interface member 500 and Figure 8G shows the fluid collection and sensor device 1150 completely engaged with the tissue interface member 1150 such that fluid 1250 in the tissue can pass through the openings 1200 in the tissue and into the fluid collection chamber 1190. A fluid collection and sensor device of this type comprises an assay element that reacts with one or more analytes, such as glucose, to provide a reading of a concentration of such one or more analytes for an individual.

Once the alignment device of the present invention is properly placed, the systems and methods of the present invention allow for new fluid collection and sensor devices to attach to the tissue interface member after poration has occurred to thereby use the same set of tissue openings formed at the location of the tissue interface member. The advantage is that the same set of openings can be used repeatedly for fluid extraction without having to make new openings. Consequently, whereas the fluid collection and sensor device may have a limited useful lifetime, new ones can be installed to use the same set of openings repeatedly for fluid extraction without having to make new openings. Similarly, for delivery applications, the same set of openings can be used for different and multiple delivery events.

According to another aspect of the present invention, a mechanism is provided to provide certain safety features and to assist in aligning an apparatus in the alignment device. These safety features may be useful to prevent tissue breaching, fluid extraction and/or substance delivery if the attachment of the apparatus device is not proper.

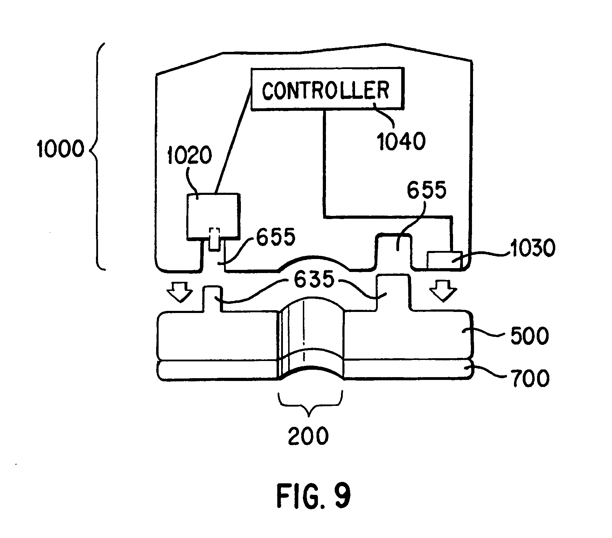

Figure 9 shows the tissue interface member engaging portion of the apparatus 1000 having at least one female member 655 allowing it to matingly engage with at least one complementary male engaging member 635 on the tissue interface member 500. The apparatus is, for example, a laser beam device of the type referred to in the above-mentioned provisional application. However, this feature may be useful in a type of apparatus that is to be operated only when properly in position in an alignment device. A sensor 1020 is provided in the apparatus 1000 that is positioned in proximity to a female member such that it is mechanically or electrically tripped when engaged by the at least one male member 635 on the tissue interface member 500. The sensor 1020 is also electrically coupled to a controller 1040. The sensor 1020 is, for example, a switch that is closed when engaged by the male member 635 on the tissue interface member 650 when the apparatus 1000 is properly engaged in the tissue interface member 500. When the switch 1020 is closed, an enable signal is coupled to the controller 1040 (or a circuit is completed and detected by the controller 1040) which will in response, enable operation of the apparatus. While the apparatus 1000 is properly mated to the tissue interface member 500, the apparatus is fully enabled and may be activated by a control button (or other user control or automatic control mechanism) to interact with the surface of the tissue. In the embodiment shown in Figure 9, the apparatus 1000 interacts with the surface of the tissue through the opening 200 of the tissue interface member 500. As an additional optional feature, a pressure (force) sensor 1030 is also provided that is responsive to upward pressure from the tissue interface member 500 when the apparatus 1000, such a laser beam device, is pressed downward. Sufficient downward pressure of the apparatus 1000 against the tissue interface member may be a prerequisite to enabling activation or actual activation of the apparatus. In this way, the apparatus will not be activated unless the switch 1020 detects proper engagement in the tissue interface member 500 and the pressure sensor 1030 detects that sufficient downward force is being applied to the apparatus 1000.

According to one aspect, the present invention is directed to an alignment device for aligning at least one apparatus with respect to a surface of a tissue, comprising a tissue interface member suitable for positioning on the surface of the tissue and mating with the apparatus to maintain alignment of the apparatus during an operation of the apparatus.

According to another aspect, the present invention is directed to a system comprising: a tissue interface member suitable for positioning on the surface of the tissue; a tissue breaching apparatus that mates with the tissue interface member to achieve a desired alignment with the surface of the tissue; and a sensor device capable of mating to the tissue interface member when the tissue breaching device is not mated to the tissue interface member to achieve alignment with an ablated site of the tissue, wherein the sensor device detects a characteristic of a biological fluid collected from the ablated site of the tissue. The tissue breaching device may be any device that mechanically breaches the tissue, a heatable element device that thermally ablates the tissue, and an energy emitter device capable of emitting energy that is directly absorbed by the tissue. Alternatively, the tissue breaching device cooperates with an energy emitter device that cooperates with an energy absorbing layer positioned on, or a part of, the tissue interface member.

The present invention can be used for a method for detecting a characteristic of a biological tissue, comprising the steps of: placing a tissue interface member at a desired position onto the surface of the tissue; mating a tissue breaching apparatus to the tissue interface member to achieve alignment with the surface of the tissue; activating the tissue breaching apparatus; detaching the tissue breaching apparatus from the tissue interface member; and mating a sensor device to the tissue interface member to achieve alignment with a breached tissue site.

The present invention also is directed to a sensor device for sensing a characteristic of a biological fluid collected from a tissue, comprising: a housing; at least one opening in the housing to collect biological fluid from the tissue; at least one alignment member suitable for mating with a complementary alignment member of a tissue interface member positioned on a surface of the tissue for aligning the at least one opening in the housing with a predetermined surface portion of the tissue.

Similarly, the present invention is directed to an energy emitter apparatus comprising: an energy source for emitting energy suitable for absorption by an energy absorbing layer positioned in substantial contact with a surface of a tissue; and at least one alignment member suitable for mating with at least one complementary alignment member of a tissue interface member positioned on a surface of the tissue for aligning the energy emitted by the energy source with the energy absorbing layer.

The above description is intended by way of example only.

## Claims

1. An alignment system comprising:
a. a tissue interface member (100, 500) suitable for positioning on the surface of the tissue;
b. a plurality of mating devices, including:
i. a tissue breaching device (1150) having an energy emitter (1010) capable of causing one or more points of breach in the surface of the tissue;
ii a biological fluid detector;
iii a substance delivery device adapted to deliver a substance into a tissue via a breach in the surface of the tissue;
whereby each of the mating devices (1000) is configured to exactly and repeatably align, via the tissue interface member (100, 500) to the tissue, each time a device is mated with the tissue interface member (100, 500).

2. The system of claim 1, further comprising an energy absorbing layer (400), which is interposed between the tissue interface member (100, 500) and the surface of the tissue, that is responsive to energy from the tissue breaching device (1000) and conducts heat to the surface of the tissue to ablate the tissue.

3. The system of claim 2, wherein the energy absorbing layer (400) has a first and second side, and whereby an adhesive material disposed on the second side adheres the energy absorbing layer to the surface of the tissue.

4. The system of claim 2, wherein the tissue interface member further comprises an inner periphery, and whereby a separation line, located on the energy absorbing layer and adjacent to the inner periphery, facilitates the removal of the energy absorption layer from the tissue interface member after tissue ablation.

5. The system of claim 1, wherein the tissue interface member further comprises at least one clip (600) that mates with a surface of the device (1000) being mated with the tissue interface member (100, 500) and holds said device in alignment with the surface of the tissue.

6. The system of claim 5, wherein the clip (600) is biased to hold the aligned device (1000) under tension.

7. The system of claim 1, wherein the tissue interface member has an exterior and an interior surface, and whereby at least one of the surfaces of the tissue interface member is adapted to mate with the device in a repeatable alignment position relative to the surface of the tissue.

8. The system of claim 1, wherein the tissue interface member (100, 500) further comprises at least one magnetic surface portion that mates with at least one complementary magnetic surface portion on the aligned device whereby the alignment of the device relative to the surface of the tissue is maintained.

9. The system of claim 1, wherein the tissue interface member (100, 500) further comprises an adhesive element (700) that allows the tissue interface member to be attached to the surface of the tissue.

10. The system of claim 1, wherein the tissue breaching device (1000) further comprises a controller (1040) and a sensor (1020) that is coupled to the controller, whereby the energy emitter (1010) is activated by the controller when the sensor detects that the energy emitter is in mated to the tissue interface member.

11. The system of claim 10, wherein the sensor (1020) is a switch that is closed by an element on the tissue interface member (100, 500) when the energy emitter device is properly mated to the tissue interface member.

12. The system of any of the preceding claims, wherein the energy emitter apparatus further comprises a pressure sensor (1030) responsive to sufficient pressure from engagement of the tissue breaching device (1000) with the tissue interface member (100, 500), and whereby the controller activates the energy emitter when both the switch is closed and the pressure sensor detects sufficient pressure.

13. The system of claim 1, wherein the energy emitter of the tissue breaching device (1060) mechanically forms at least one breach in the tissue.

14. The system of claim 1, wherein the energy emitter of the tissue breaching device (1042) comprises a heatable element (1045), and whereby the element thermally ablates the tissue to form at least one breach therein.

15. The system of claim 14, further comprising an energy absorbing layer (400) attached to the tissue interface member, wherein the energy absorbing layer is responsive to heat energy from the tissue breaching device and conducts heat to the surface of the tissue to ablate the tissue.

16. The system of claim 15, wherein the energy absorbing layer (400) is arranged to be simultaneously removed from the surface of the tissue upon detachment of the tissue breaching device.

17. The system of claim 1, wherein the detector device draws biological fluid from one or more breaches in the surface of the tissue.

18. The system of claim 1, wherein the substance delivery device further comprises a controller (1040) and a sensor (1020) that is coupled to the controller, whereby the substance delivery device is activated by the controller when the sensor detects that the substance delivery device is mated to the tissue interface member.

19. The system of claim 18, wherein the sensor is a switch that is closed by an element on the tissue interface member when the substance delivery device is properly mated to the tissue interface member.

20. The system of any of the preceding claims, wherein the substance delivery device further comprises a pressure sensor responsive to sufficient pressure from engagement of the substance delivery device with the tissue interface device, and whereby the controller activates the delivery device when both the switch is closed and the pressure sensor detects sufficient pressure.

## Patentansprüche

1. Ausrichtungssystem umfassend:
a) ein Gewebe-Schnittstellenelement (100,500), welches zur Anordnung auf der Oberfläche des Gewebes geeignet ist;
b) mehrere passende Vorrichtungen, einschließlich:
i) einer Gewebebrechvorrichtung (1150) mit einer Energiequelle (1010), die dazu in der Lage ist, einen oder mehrere Durchbruchpunkte in der Oberfläche des Gewebes zu erzeugen;
ii) eines Detektors für biologische Flüssigkeit;
iii) einer Substanzabgabevorrichtung, die dazu eingerichtet ist, eine Substanz in das Gewebe über einen Durchbruch in der Oberfläche des Gewebes abzugeben;
wobei jede der passenden Vorrichtungen (1000) dazu eingerichtet ist, genau und wiederholbar über das Gewebe-Schnittstellenelement (100, 500) zu dem Gewebe ausgerichtet zu werden, jedes Mal wenn eine Vorrichtung in das Gewebe-Schnittstellenelement (100, 500) eingreift.

2. System nach Anspruch 1, ferner umfassend eine Energie absorbierende Schicht (400), die zwischen dem Gewebe-Schnittstellenelement (100, 500) und der Oberfläche des Gewebes angeordnet ist, die auf Energie von der Gewebebrechvorrichtung (1000) reagiert und Wärme an die Oberfläche des Gewebes leitet, um das Gewebe zu abladieren.

3. System nach Anspruch 2, wobei die Energie absorbierende Schicht (400) eine erste und eine zweite Seite aufweist und wobei ein auf der zweiten Seite angeordnetes haftendes Material die Energie absorbierende Schicht an der Oberfläche des Gewebes festhält.

4. System nach Anspruch 2, wobei das Gewebe-Schnittstellenelement ferner einen inneren Umfang aufweist und eine Separationslinie, die auf der Energie absorbierenden Schicht und neben dem inneren Umfang angeordnet ist, die Entfernung der Energieabsorbierenden Schicht von dem Gewebe-Schnittstellenelement nach der Gewebeablation erleichtert.

5. System nach Anspruch 1, wobei das Gewebe-Schnittstellenelement ferner wenigstens eine Halteschelle (600) aufweist, die zu einer Oberfläche der Vorrichtung (1000) passt, die in das Gewebe-Schnittstellenelement (100, 500) eingreift und diese Vorrichtung zu der Oberfläche des Gewebes ausrichtet.

6. System nach Anspruch 5, wobei die Halteschelle (600) federnd ausgestaltet ist, um die ausgerichtete Vorrichtung (1000) unter Spannung zu halten.

7. Das System nach Anspruch 1, wobei das Gewebe-Schnittstellenelement eine äußere und eine innere Oberfläche aufweist, wobei wenigstens eine der Oberflächen des Gewebe-Schnittstellenelements dazu eingerichtet ist, in die Vorrichtung in einer wiederholbaren Ausrichtungsposition in Bezug auf die Oberfläche des Gewebes einzugreifen.

8. System nach Anspruch 1, wobei das Gewebe-Schnittstellenelement (100, 500) ferner wenigstens einen magnetischen Oberflächenabschnitt aufweist, der zu wenigstens einem komplementären magnetischen Oberflächenabschnitt auf der ausgerichteten Vorrichtung passt, womit die Ausrichtung der Vorrichtung in Bezug auf die Oberfläche des Gewebes gehalten wird.

9. System nach Anspruch 1, wobei das Gewebe-Schnittstellenelement (100, 500) ferner ein Haftelement (700) umfasst, welches dem Gewebe-Schnittstellenelement erlaubt, auf der Oberfläche des Gewebes befestigt zu werden.

10. System nach Anspruch 1, wobei die Gewebebrechvorrichtung (1000) ferner eine Steuerung (1040) und einen Sensor (1020) umfasst, der mit der Steuerung gekoppelt ist, wobei die Energiequelle (1010) durch die Steuerung aktiviert wird, wenn der Sensor detektiert, dass die Energiequelle in das Gewebe-Schnittstellenelement eingreift.

11. System nach Anspruch 10, wobei der Sensor (1020) ein Schalter ist, der durch ein Element auf dem Gewebe-Schnittstellenelement (100, 500) geschlossen wird, wenn die Energiequellenvorrichtung richtig in das Gewebe-Schnittstellenelement eingreift.

12. System nach einem der vorhergehenden Ansprüche, wobei die Energiequellenvorrichtung ferner einen Drucksensor (1030) umfasst, der auf ausreichenden Druck vom Eingreifen der Gewebebrechvorrichtung (1000) in das Gewebe-Schnittstellenelement (100, 500) reagiert, und wobei die Steuerung die Energiequelle aktiviert, wenn sowohl der Schalter geschlossen ist als auch der Drucksensor ausreichenden Druck detektiert.

13. System nach Anspruch 1, wobei die Energiequelle der Gewebebrechvorrichtung (1060) mechanisch wenigstens einen Durchbruch im Gewebe bildet.

14. System nach Anspruch 1, wobei die Energiequelle der Gewebebrechvorrichtung (1042) ein heizbares Element (1045) aufweist und wobei das Element das Gewebe thermisch abladiert, um darin wenigstens einen Durchbruch zu bilden.

15. System nach Anspruch 14, welches ferner eine energieabsorbierende Schicht (400) aufweist, die an dem Gewebe-Schnittstellenelement befestigt ist, wobei die energieabsorbierende Schicht auf Wärmeenergie von der Gewebebrechvorrichtung reagiert und die Wärme an die Oberfläche des Gewebes leitet, um das Gewebe zu abladieren.

16. System nach Anspruch 15, wobei die energieabsorbierende Schicht (400) dazu eingerichtet ist, bei dem Abnehmen der Gewebebrechvorrichtung simultan von der Oberfläche des Gewebes entfernt zu werden.

17. System nach Anspruch 1, wobei die Detektorvorrichtung biologische Flüssigkeit aus einem oder mehreren Durchbrüchen in der Oberfläche des Gewebes zieht.

18. System nach Anspruch 1, wobei die Substanzabgabevorrichtung ferner eine Steuerung (1040) und einen Sensor (1020) umfasst, der mit der Steuerung gekoppelt ist, wobei die Substanzabgabevorrichtung durch die Steuerung aktiviert wird, wenn der Sensor detektiert, dass die Substanzabgabevorrichtung in das Schnittstellenelement eingreift.

19. System nach Anspruch 18, wobei der Sensor ein Schalter ist, der durch ein Element auf dem Gewebe-Schnittstellenelement geschlossen wird, wenn die Substanzabgabevorrichtung richtig in das Gewebe-Schnittstellenelement eingreift.

20. System nach einem der vorhergehenden Ansprüche, wobei die Substanzabgabevorrichtung ferner einen Drucksensor aufweist, der auf ausreichenden Druck vom Eingreifen der Substanzabgabevorrichtung in die Gewebe-Schnittstellenvorrichtung reagiert, wobei die Steuerung die Abgabevorrichtung aktiviert, wenn sowohl der Schalter geschlossen ist als auch der Drucksensor einen ausreichenden Druck detektiert.

## Revendications

1. Un système d'alignement, comprenant:
a. un organe d'interface (100, 500) avec un tissu (ensemble de cellules) convenant pour un positionnement sur la surface du tissu;
b. une pluralité de dispositifs d'adaptation, comprenant:
i. un dispositif de fissuration de tissu (1150) ayant un émetteur d'énergie (1010) capable de provoquer un ou plusieurs points de fissuration dans la surface du tissu;
ii. un détecteur de fluide biologique;
iii. un dispositif de fourniture de substance, apte à délivrer une substance dans un tissu via une fissure ménagée dans la surface du tissu;
de manière que chacun des dispositifs d'adaptation (1000) soit configuré pour s'aligner de façon exacte et répétable, via l'organe d'interface avec le tissu (100, 500) vis-à-vis du tissu, chaque fois qu'un dispositif est accouplé à l'organe d'interface de tissu (100, 500).

2. Le système selon la revendication 1, comprenant en outre une couche d'absorption d'énergie (400), qui est interposée entre l'organe d'interface avec le tissu (100, 500) et la surface du tissu, qui réagit à l'énergie provenant du dispositif de fissuration de tissu (1000) et conduit la chaleur vers la surface du tissu pour produire une ablation du tissu.

3. Le système selon la revendication 2, dans lequel la couche d'absorption d'énergie (400) présente une première et une deuxième face, et de manière qu'un matériau adhésif, disposé sur la deuxième face, adhère à la couche d'absorption d'énergie sur 1a surface du tissu.

4. Le système selon la revendication 2, dans lequel l'organe d'interface avec le tissu comprend en outre une périphérie interne, et de manière qu'une ligne de séparation, située sur la couche d'absorption d'énergie et adjacente à la périphérie interne, facilite l'enlèvement de la couche d'absorption d'énergie de l'organe d'interface avec le tissu, après l'ablation du tissu.

5. Le système selon la revendication 1, dans lequel l'organe d'interface avec le tissu comprend en outre au moins une attache (600), qui s'adapte à une surface du dispositif (1000) adaptée à l'organe d'interface (100, 500) avec le tissu et maintient ledit dispositif en alignement avec la surface du tissu.

6. Le système selon la revendication 5, dans lequel l'attache (600) est sollicitée pour maintenir sous tension mécanique le dispositif (1000) ayant été aligné.

7. Le système selon la revendication 1, dans lequel l'organe d'interface avec le tissu présente une surface extérieure et une surface intérieure, et de manière qu'au moins l'une des surfaces de l'organe d'interface avec le tissu soit apte à s'accoupler au dispositif, en une position d'alignement répétable par rapport à la surface du tissu.

8. Le système selon la revendication 1, dans lequel l'organe d'interface avec le tissu (100, 500) comprend en outre au moins une partie de surface magnétique, qui s'accouple à au moins une partie de surface magnétique complémentaire située sur le dispositif aligné, de manière que l'alignement du dispositif par rapport à la surface du tissu soit conservé.

9. Le système selon la revendication 1, dans lequel l'organe d'interface avec le tissu (100, 500) comprend en outre un élément adhésif (700), permettant à l'organe d'interface avec la tissu d'être fixé sur la surface du tissu.

10. Le système selon la revendication 1, dans lequel le dispositif (1000) de fissuration du tissu comprend en outre un contrôleur (1040) et un capteur (1020) couplé au contrôleur, de manière que l'émetteur d'énergie (1010) soit activé par le contrôleur, lorsque le capteur détecte que l'émetteur d'énergie est en situation d'accouplement avec l'organe d'interface avec le tissu.

11. Le système selon la revendication 10, dans lequel le capteur (1020) est un interrupteur qui est fermé par un élément situé sur l'organe d'interface avec le tissu (100, 500) lorsque le dispositif émetteur d'énergie est correctement accouplé sur l'organe d'interface de tissu.

12. Le système selon l'une quelconque des revendications précédentes, dans lequel le dispositif émetteur d'énergie comprend en outre un capteur de pression (1030), réagissant à une pression suffisante, de mise en contact du dispositif de fissuration du tissu (1000) avec l'organe d'interface avec le tissu (100, 500), et de manière que le contrôleur active l'émetteur d'énergie, lorsque à la fois l'interrupteur est fermé et le capteur de pression détecte une pression suffisante.

13. Le système selon la revendication 1, dans lequel l'émetteur d'énergie du dispositif de fissuration du tissu (1060) forme mécaniquement au moins une fissure dans le tissu.

14. Le système selon la revendication 1, dans lequel l'émetteur d'énergie du dispositif de fissuration du tissu (1042) comprend un élément apte à être chauffé (1045), et de manière que l'élément accomplisse une ablation thermique du tissu, afin d'y former au moins une fissure.

15. Le système selon la revendication 14, comprenant en outre une couche d'absorption d'énergie (400) attachée à l'organe d'interface avec le tissu, dans lequel la couche d'absorption d'énergie réagit à l'énergie thermique provenant du dispositif de fissuration du tissu et conduit la chaleur vers la surface du tissu pour procéder à l'ablation du tissu.

16. Le système selon la revendication 15, dans lequel la couche d'absorption d'énergie (400) est agencée pour être simultanément enlevée de la surface du tissu lors du détachement du dispositif de fissuration du tissu.

17. Le système selon la revendication 1, dans lequel le dispositif détecteur aspire du fluide biologique d'une ou plusieurs fissures ménagées dans la surface du tissu.

18. Le système selon la revendication 1, dans lequel le dispositif de fourniture de substance comprend en outre un contrôleur (1040) et un capteur (1020), couplé au contrôleur, de manière que le dispositif de fourniture de substance soit activé par le contrôleur, lorsque le capteur détecte que le dispositif de fourniture de substance est accouplé à l'organe d'interface avec le tissu.

19. Le système selon la revendication 18, dans lequel le capteur est un interrupteur qui est fermé par un élément prévu sur l'organe d'interface avec le tissu lorsque le dispositif de fourniture de substance est correctement accouplé à l'organe d'interface avec le tissu.

20. Le système selon l'une quelconque des revendications précédentes, dans lequel le dispositif de fourniture de substance comprend en outre un capteur de pression réagissant à une pression suffisante issue de la mise en contact du dispositif de fourniture de substance avec le dispositif d'interface de tissu, et de manière que le contrôleur active le dispositif de fourniture lorsque, à la fois, l'interrupteur est fermé et que le capteur de pression détecte une pression suffisante.
